(19) Europäisches Patentamt
European Patent Office
Office européen des brevets

(11) Veröffentlichungsnummer: **0 256 990 A1**

(12) # EUROPÄISCHE PATENTANMELDUNG

(21) Anmeldenummer: **87810462.9**

(22) Anmeldetag: **14.08.87**

(51) Int. Cl.⁴: **C 07 D 499/00**
A 61 K 31/43
// C07D213/24

(30) Priorität: **20.08.86 CH 3346/86**

(43) Veröffentlichungstag der Anmeldung:
**24.02.88 Patentblatt 88/08**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Schneider, Peter, Dr.**
**Bäumliackerstrasse 8**
**CH-4103 Bottmingen (CH)**

(54) **Pyridinio-Verbindungen.**

(57) Verbindungen der Formel

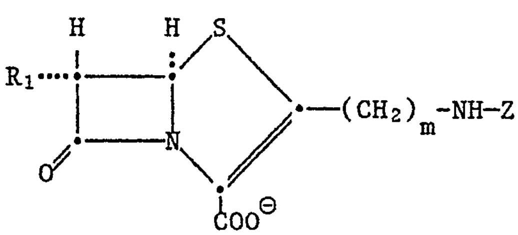

(I),

worin $R_1$ für Hydroxymethyl oder 1-Hydroxyäthyl steht, Z für einen Rest

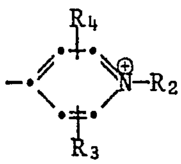

oder

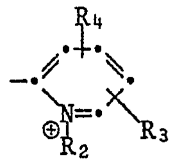

steht, worin $R_2$ gegebenenfalls substituiertes Niederalkyl, Niederalkenyl, gegebenenfalls substituiertes Phenyl, Pyridyl oder veräthertes Hydroxy bedeutet, $R_3$ und $R_4$ unabhängig voneinander für Wasserstoff, gegebenenfalls substituiertes Niederalkyl, gegebenenfalls funktionell abgewandeltes Carboxyl, veräthertes Hydroxy, verestertes Hydroxy oder gegebenenfalls substituiertes Amino stehen und m eine ganze Zahl von 1 bis 4 ist, und Salze von Verbindungen der Formell I haben antibiotische Wirksamkeit. Die Verbindungen der Formel I werden nach an sich bekannten Verfahren hergestellt.

EP 0 256 990 A1

**Beschreibung**

Pyridino-Verbindungen

Die Erfindung betrifft Penem-Verbindungen der Formel

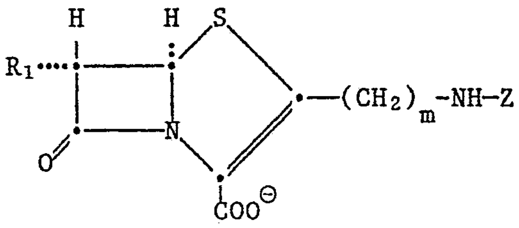

(I),

worin $R_1$ für Hydroxymethyl oder 1-Hydroxyäthyl steht, Z für einen Rest

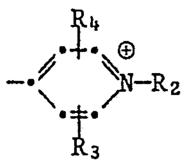

oder

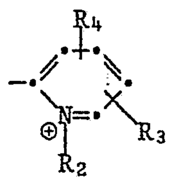

steht, worin $R_2$ gegebenenfalls substituiertes Niederalkyl, Niederalkenyl, gegebenenfalls substituiertes Phenyl, Pyridyl oder veräthertes Hydroxy bedeutet, $R_3$ und $R_4$ unabhängig voneinander für Wasserstoff, gegebenenfalls substituiertes Niederalkyl, gegebenenfalls funktionell abgwandeltes Carboxyl, veräthertes Hydroxy, verestertes Hydroxy oder gegebenenfalls substituiertes Amino stehen und m eine ganze Zahl von 1 bis 4 ist, Salze von Verbindungen der Formel I, Verfahren zur Herstellung von Verbindungen der Formel I, pharmazeutische Präparate, die solche Verbindungen enthalten, und ihre Verwendung zur Herstellung von pharmazeutischen Präparaten oder als Arzneimittelwirkstoffe.

Die vor- und nachstehend verwendeten Definitionen haben im Rahmen der vorliegenden Beschreibung vorzugsweise die folgenden Bedeutungen:

Substituiertes Niederalkyl als Substituent $R_2$, $R_3$ und $R_4$ ist beispielsweise durch Hydroxy, veräthertes oder verestertes Hydroxy, Carboxyl, funktionell abgewandeltes Carboxyl, Amino, Azido, gegebenenfalls substituiertes Phenyl oder Oxo substituiertes Niederalkyl.

Substituiertes Phenyl ist insbesondere durch Niederalkyl, Niederalkoxy, Halogen, Cyano und/oder Carboxy mono- oder auch disubstituiertes Phenyl.

Veräthertes Hydroxy ist beispielsweise Niederalkoxy.

Verestertes Hydroxy ist z.B. Halogen Niederalkanoyloxy oder Carbamoyloxy.

Funktionell abgewandeltes Carboxyl ist z.B. verestertes oder amidiertes Carboxyl, wie Niederalkoxycarbonyl, Carbamoyl, N-Niederalkyl- oder N,N-Diniederalkylcarbamoyl, oder Cyano.

Substituiertes Amino ist beispielsweise niederalkyliertes Amino, wie Niederalkylamino oder Diniederalkylamino, Niederalkylenamino oder acyliertes Amino, z.B. Niederalkanoylamino.

In der vorliegenden Beschreibung bedeutet der im Zusammenhang mit Definitionen von Gruppen bzw. Verbindungen verwendete Ausdruck "nieder", dass die entsprechenden Gruppen bzw. Verbindungen, sofern nicht ausdrücklich anders definiert, 1 bis 7, bevorzugt 1 bis 4, Kohlenstoffatome enthalten.

Niederalkyl ist z.B. n-Propyl, Isopropyl, n-Butyl, sek.-Butyl, Isobutyl und in erster Linie Methyl oder Aethyl.

Niederalkenyl hat 2 bis 5 Kohlenstoffatome und ist z.B. Allyl, Methallyl oder Crotonyl.

Halogen als Substituent eines Niederalkyl- oder Phenylrestes ist Fluor, Chlor, Brom oder Jod, während Halogen als Pyridinring-Substituent $R_3$ oder $R_4$ insbesondere Chlor oder Brom ist.

Niederalkanoyloxy ist z.B. Formyloxy oder Acetyloxy.

Niederalkoxy ist z.B. n-Propyloxy, n-Butyloxy und in erster Linie Methoxy oder Aethoxy.

Niederalkoxycarbonyl ist z.B. Methoxycarbonyl oder Aethoxycarbonyl.

N-Niederalkylcarbamoyl ist z.B. N-Methyl- oder N-Aethylcarbamoyl, während N,N-Diniederalkylcarbamoyl z.B. N,N-Dimethylcarbamoyl ist.

Niederalkylamino ist z.B. Methylamino oder Aethylamino, während Diniederalkylamino z.B. Dimethylamino oder Diäthylamino ist.

Niederalkylenamino ist insbesondere $C_4$-$C_5$-Alkylenamino und bedeutet beispielsweise Pyrrolidino oder Piperidino.

Niederalkanoylamino ist z.B. Formylamino, Acetamino oder Propionylamino.

Pyridyl ist z.B. 2-, 3- und insbesondere 4-Pyridyl.

In bevorzugten Verbindungen der Formel I steht $R_1$ für 1-Hydroxyäthyl, insbesondere (1R)-1-Hydroxyäthyl,

und m für 1 oder 2. Bevorzugte Pyridinio-Reste

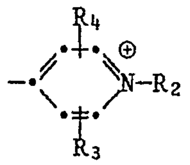

in Verbindungen der Formel I sind 1-Niederalkyl-, z.B. 1-Methyl-oder 1-Aethyl-, 1-Carboxyniederalkyl-, wie 1-Carboxymethyl- oder 1-(2-Carboxyäthyl)-, 1-Carbamoylniederalkyl-, 1-N,N-Diniederalkyl-carbamoylniederalkyl- oder 1-Niederalkoxycarbonylniederalkyl-, wie 1-Carbamoylmethyl-, 1-N,N-Dimethylcarbamoylmethyl-, 1-Methoxy-carbonylmethyl- oder 1-Aethoxycarbonylmethyl-, 1-Cyanoniederalkyl-, wie 1-Cyanomethyl, 1-Phenylniederalkyl-, wie 1-Benzyl-, 1-Pyridyl-, wie 1-(4-Pyridyl)-, 1-Acetonyl-, ferner 1-Niederalkyl-2-niederalkoxycarbonyl-, wie 1-Methyl-2-äthoxycarbonyl-, 1-Niederalkyl-2-carboxy-, wie 1-Methyl-2-carboxy-, 1-Niederalkyl-2-cyano-, wie 1-Methyl-2-cyano-, 1-Niederalkyl-2-carbamoyl-, wie 1-Methyl-2-carbamoyl-, 1-Niederalkyl-2-N,N-diniederalkylcarbamoyl-, wie 1-Methyl-2-N,N-dimethylcarbamoyl-, 1-Niederalkyl-2-hydroxynieder-alkyl-, wie 1-Methyl-2-hydroxymethyl-, 1-Niederalkyl-2-aminoniederalkyl-, wie 1-Methyl-2-aminomethyl-, 1-Niederalkyl-2-carboxyniederalkyl-, wie 1-Methyl-2-carboxymethyl-, 1-Niederalkyl-2-niederalkoxycarbonylniederalkyl-, wie 1-Methyl-2-methoxy(oder äthoxy)-carbonylmethyl-, und 1-Niederalkyl-2-cyanoniederalkyl-, wie 1-Methyl-2-cyanomethyl(oder cyanoäthyl)-4-pyridinio.

Bevorzugte Pyridinio-Reste

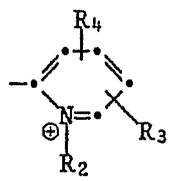

in Verbindungen der Formel I sind 1-Niederalkyl-, z.B. 1-Methyl-, 1-Niederalkyl-5-carbamoyl-, wie 1-Methyl-5-carbamoyl-, 1-Niederalkyl-5-hydroxyniederalkyl-, wie 1-Methyl-5-hydroxymethyl-, 1- Niederalkyl-5-carbamoyloxyniederalkyl-, wie 1-Methyl-5-carbamoyloxy-methyl-, und 1-Niederalkyl-5-aminoniederalkyl-, wie 1-Methyl-5-aminomethyl-2-pyridinio.

Salze von erfindungsgemässen Verbindungen sind in erster Linie pharmazeutisch annehmbare, nicht-toxische Salze von Verbindungen der Formel I. Solche Salze werden beispielsweise von den in Verbindungen der Formel I vorhandenen sauren Gruppen, z.B. Carboxylgruppen (als Substituenten der Reste $R_2$, $R_3$ oder $R_4$) gebildet und sind in erster Linie Metall- oder Ammoniumsalze, wie Alkalimetall- und Erdalkali-metall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin, Tris-(2-hydroxyäthyl)-amin oder 2-Amino-1,3-dihydroxy-2-hydroxymethyl-propan ("Tris"), basischen aliphatischen Estern von Carbonsäuren, z.B. 4-Aminobenzoesäure-2-diäthylaminoäthylester, Niederalkylenaminen, z.B. 1-Aethyl-piperidin, Cycloalkylaminen, z.B. Dicyclohexylamin, oder Benzylaminen, z.B. N,N'-Dibenzyläthylendiamin, Dibenzylamin oder N-Benzyl-ß-phenäthylamin. Verbindungen der Formel I mit einer basischen Gruppe, z.B. mit einer Aminogruppe (als Substituent der Reste $R_3$ oder $R_4$), können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Erfindung betrifft insbesondere Verbindungen der Formel I, worin $R_1$ Hydroxymethyl oder (1R)-1-Hydroxyäthyl ist, Z für einen Rest

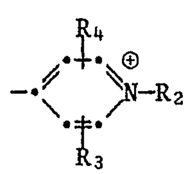 oder 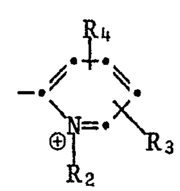

steht, worin $R_2$ Niederalkyl, durch Hydroxy, Halogen, Niederalkanoyloxy, Carbamoyloxy, Niederalkoxy,

Carboxyl, Niederalkoxy-carbonyl, Carbamoyl, N-Niederalkyl- oder N,N-Diniederalkylcarbamoyl, Cyano, Amino, Azido, Phenyl, durch Niederalkyl, Niederalkoxy, Halogen, Cyano und/oder Carboxy substituiertes Phenyl oder Oxo substituiertes Niederalkyl; Niederalkenyl, Phenyl, durch Niederalkyl, Niederalkoxy, Halogen, Cyano und/oder Carboxy substituiertes Phenyl, Pyridyl oder Niederalkoxy bedeutet, $R_3$ und $R_4$ unabhängig voneinander für Wasserstoff, Niederalkyl, durch Hydroxy, Halogen, Niederalkanoyloxy, Carbamoyloxy, Niederalkoxy, Carboxy, Niederalkoxycarbonyl, Carbamoyl, N-Niederalkyl- oder N,N-Diniederalkyl-carbamoyl, Cyano, Amino, Azido, Phenyl, durch Niederalkyl, Niederalkoxy, Halogen, Cyano und/oder Carboxy substituiertes Phenyl oder Oxo substituiertes Niederalkyl; Carboxyl, Niederalkoxycarbonyl, Carbamoyl, N-Niederalkyl- oder N,N-Diniederalkylcarbamoyl, Cyano, Niederalkoxy, Halogen, Niederalkanoyloxy, Carbamoyloxy, Amino, Niederalkylamino, Diniederalkylamino, Niederalkylenamino oder Niederalkanoylamino stehen, m eine ganze Zahl von 1 bis 4 ist, und Salze von Verbindungen der Formel I.

Die Erfindung betrifft hauptsächlich Verbindungen der Formel I, worin $R_1$ (1R)-1-Hydroxyäthyl ist, Z für einen Rest

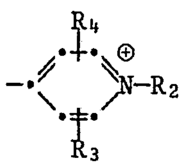

steht, worin $R_2$ Niederalkyl, Acetonyl oder durch Carboxy, Niederalkoxycarbonyl, Carbamoyl, N,N-Diniederalkylcarbamoyl oder Phenyl substituiertes Niederalkyl bedeutet, $R_3$ Wasserstoff bedeutet, $R_4$ für Wasserstoff, durch Hydroxy, Carboxy, Cyano oder Amino substituiertes Niederalkyl; Carboxy, Carbamoyl oder Cyano steht, und m 1 oder 2 ist, und Salze von Verbindungen der Formel I.

Die Erfindung betrifft in erster Linie Verbindungen der Formel I, worin $R_1$ (1R)-1-Hydroxyäthyl ist, Z für einen Rest

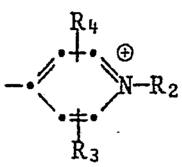

steht, worin $R_2$ Niederalkyl oder durch Carbamoyl substituiertes Niederalkyl ist, $R_3$ Wasserstoff ist und $R_4$ Wasserstoff oder durch Hydroxy substituiertes Niederalkyl bedeutet und m 1 ist, und Salze von Verbindungen der Formel I.

Die Erfindung betrifft vor allem die in Beispielen genannten Verbindungen der Formel I.

Die Verbindungen der vorliegenden Erfindung können nach an sich bekannten Verfahren hergestellt werden.

Die neuen Verbindungen werden z.B. hergestellt, in dem man eine Penem-Verbindungen der Formel I

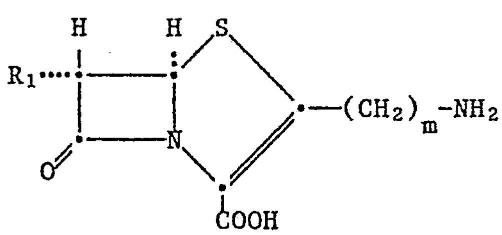

(II),

worin $R_1$ und m die unter Formel I angegebenen Bedeutungen haben, mit einer Pyridinium-Verbindungen der Formel

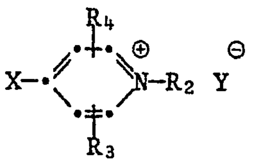 (III) oder 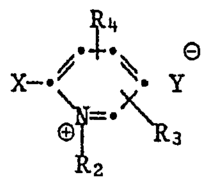 (IIIa),

worin $R_2$, $R_3$ und $R_4$ die unter Formel I angegebenen Bedeutungen haben, wobei funktionelle Gruppen in diesen Resten gegebenenfalls in geschützter Form vorliegen, X eine Abgangsgruppe ist und $Y^{\ominus}$ für ein Anion steht, umsetzt und, falls erforderlich, geschützte funktionelle Gruppen in den Resten $R_2$, $R_3$ und/oder $R_4$ in die freien funktionellen Gruppen überführt, und, wenn erwünscht, eine erhältliche freie Verbindung der Formel I in ein Salz überführt.

Die Abgangsgruppe X ist insbesondere eine leicht abspaltbare durch Amino ersetzbare Abgangsgruppe, wie niederes Halogen, z.B. Fluor oder Chlor, Sulfonyloxy, wie insbesondere gegebenenfalls durch Halogen substituiertes Niederalkansulfonyloxy, z.B. Methan- oder Trifluormethansulfonyloxy, oder gegebenenfalls durch Halogen oder Niederalkyl substituiertes Benzolsulfonyloxy, z.B. Benzolsulfonyl-oxy oder 4-Brombenzolsulfonyloxy, oder Niederalkoxysulfonyloxy, wie Methoxysulfonyloxy. Bevorzugte Abgangsgruppen X sind Fluor und Chlor.

Das Anion $Y^{\ominus}$ ist insbesondere ein Anion, welches eine gute Löslich-keit der Verbindung der Formel III bzw. IIIa im Reaktionsmedium begüngstigt. Solche Anionen sind beispielsweise Halogen-Anionen, wie Chlorid, Bromid und Jodid, ferner Hydrogensulfat oder Niederalkylsulfat, wie Methylsulfat.

Schutzgruppen in der Verbindung der Formel III bzw. IIIa sind insbesondere solche, die sich auf schonende Weise, d.h. ohne dass unerwünschte Nebenreaktionen stattfinden, beispielsweise schonend reduktiv oder solvolytisch, abspalten lassen.

Bevorzugte Schutzgruppen für Carboxylgruppen in Resten $R_2$, $R_3$ und/oder $R_4$ sind beispielsweise gegebenenfalls durch Niederalkyl, Niederalkoxy, Halogen oder Nitro substituiertes Benzyl, wie 4-Methoxy- oder 4-Nitrobenzyl, ferner auch Niederalkenyl, wie Allyl.

Bevorzugte Schutzgruppen für Aminogruppen in den Resten $R_3$ und/oder $R_4$ sind beispielsweise gegebenenfalls durch Niederalkoxy oder Nitro substituiertes Benzyloxycarbonyl, wie 4-Nitrobenzyloxycarbonyl, ferner Niederalkenyloxycarbonyl, wie Allyloxycarbonyl, und Azido.

Die Umsetzung der Penem-Verbindungen der Formel II mit der Pyridinium-Verbindung der Formel III bzw. IIIa erfolgt bei Raumtemperatur oder leicht erniedrigter Temperatur, z.B. bei -10° bis +30°C, bevorzugt bei etwa 0° bis 20°, in einem inerten Lösungsmittel, wie Wasser, einem cyclischen Aether, z.B. Tetrahydrofuran, Dimethylformamid oder Mischungen davon, in Gegenwart ungefähr stöchiometrischer Mengen oder eines geringen Ueberschusses an einer organischen oder anorganischen Base, wie eines tertiären Amins, z.B. Pyridin oder eines Triniederalkylamins, z.B. Triäthylamin oder Aethyl-diisopropylamin, eines Alkalimetallhydroxids, z.B. Natriumhydroxid, oder eines Alkalimetallcarbonats, z.B. Natriumcarbonat. Vorzugsweise wird der pH-Wert der Reaktionsmischung konstant bei etwa 7 bis 9 gehalten, indem man die währen der Reaktion freiwerdende Säure H-X durch kontinuierliche Zugabe einer Lösung der Base in einem der oben genannten Lösungsmittel oder Lösungsmittelgemischen neutralisiert. Im allgemeinen erfolgt die Umsetzung mit stöchiometrischen Mengen oder mit einem Ueberschuss (bis 150 % der theoretisch erforderlichen Menge) der Pyridinium-Verbindungen der Formel III bzw. IIIa.

Geschützte funktionelle Gruppen in den verfahrensgemäss erhältlichen Penem-Verbindungen werden in an sich bekannter Weise in die freien funktionellen Gruppen überführt. Durch Allyl geschützte Carboxylgruppen und/oder durch Allyloxycarbonyl geschützte Aminogruppen in Reste $R_2$, $R_3$ und/oder $R_4$ werden beispielsweise mit einem Allylgruppenakzeptor in Gegenwart von Tetrakis-triphenylphosphinpalladium in die freien Carboxyl- und/oder Aminogruppen überführt.

Geeignete Akzeptoren für Niederalkenylgruppen, wie insbesondere die Allylgruppe, sind z.B. Amine, wie insbesondere sterisch gehinderte Amine, z.B. tert.-Butylamin, Morpholin oder Thiomorpholin, aliphatische oder cycloaliphatische ß-Dicarbonylverbindungen, z.B. Acetylaceton, Acetessigsäureäthylester oder Dimedon, ($C_2$-$C_6$)-Niederalkancarbonsäuren, z.B. Essigsäure, Propionsäure oder Hexansäure, ferner Salze davon, z.B. Natriumhexanoat, sowie Triniederalkyl-, wie Tri-n-butylzinnhydrid. Bevorzugte Akzeptoren sind Dimedon und Tri-n-butylzinnhydrid.

Gegebenenfalls substituiertes Benzyloxycarbonyl oder Benzyloxycarbonylamino sowie Azido können durch Hydrogenolyse in Gegenwart eines metallischen Hydrierkatalysators, wie eines Palladium-katalysators, gespalten werden. Alle Abspaltungsreaktionen werden bevorzugt in neutralem Medium, d.h. bei ca. pH 7, durchgeführt.

Salze von Verbindungen der Formel I mit salzbildenden Gruppen können in an sich bekannter Weise hergestellt werden. So kann man Salze von Verbindungen der Formel I mit einer freien Carboxylgruppe im Rest $R_2$, $R_3$ und/oder $R_4$ z.B. durch Behandeln mit Metallverbindungen, wie Alkalimetallsalzen von geeigneten organischen Carbonsäuren, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise

stöchiometrische Mengen oder nur einen kleinen Ueberschuss des salzbildenden Mittels verwendet. Säureadditionssalze von Verbindungen der Formel I mit einer freien Amino-, Niederalkylamino-, Diniederalkylamino- oder Niederalkylenaminogruppe im Rest $R_3$ und/oder $R_4$ erhält man in üblicher Weise, z.B. durch Behandeln mit einer geeigneten Säure oder einem geeigneten Anionenaustauscherreagenz.

Bei allen nachträglichen Umwandlungen erhaltener Verbindungen der Formel I werden solche Reaktionen bevorzugt, die unter gemässigt alkalischen oder insbesondere neutralen Bedingungen erfolgen.

Die Ausgangsverbindungen der Formel II sind bekannt, beispielsweise aus der Deutschen Offenlegungsschrift Nr. 3431980, der Japanischen Patentanmeldung Nr. 56166194 [Chemical Abstracts 96, 142565u (1982)], dem Europäischen Patent Nr. 3960 und der Europäischen Patentanmeldung Nr. 82113.

Die Pyridinium-Verbindungen der Formel III bzw. IIIa sind gleichfalls bekannt, beispielsweise aus Adv. Heterocyl. Chem. 3, 1 (1964) und 22, 71 (1978) sowie J.Medicinal Chem. 25, 457 (1982), oder können in Analogie zu den dort beschriebenen Verfahren hergestellt werden.

Die Verbindungen der Formel I weisen wertvolle pharmakologische Eingenschaften auf. Insbesondere weisen sie antibakterielle Wirkungen auf. Beispielsweise sind sie in vitro gegen grampositive und gramnegative Kokken, inkl. Methicillin-resistente Kokken, z.B. Staphylococcus aureus, Staphylococcus epidermidis, Streptococcus pyrogenes, Streptococcus pneumoniae und Streptococcus faecalis, Listeria sp. und Neisseria sp. in minimalen Konzentrationen von <0,01 bis ca. 16 µg/ml, gegen gramnegative Stäbchenbakterien, wie Enterobacteriaceae, Haemophilus influenzae und Pseudomonas aeruginosa, in minimalen Konzentrationen von ca. 0,01 bis ca. 64 µg/ml und gegen Anaerobier, wie Bacteroides fragilis oder Clostridium sp. in minimalen Konzentrationen von ca. 0,01 bis ca. 2 µg/ml wirksam.

Die neuen Verbindungen können als oral oder insbesondere parenteral applizierbare antibakterielle Breitspektrum-Antibiotika, z.B. in Form von entsprechenden pharmazeutischen Präparaten, zur Behandlung von Infektionen Verwendung finden.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. z.B. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis-oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche dosen (oral oder parenteral) von etwa 300 mg bis etwa 5 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

## Experimenteller Teil

### Herstellung von Ausgangsverbindungen

A. 4-Chlor-2-hydroxymethyl-pyridin

Eine Lösung von 72 g (0,42 Mol) 4-Chlorpicolinsäuremethylester in 1 l Tetrahydrofuran wird portionenweise bei 0°-5° mit 10 g (0,26 Mol) Lithiumaluminiumhydrid versetzt und während 1,5 h bei 0°-5° nachgerührt. Durch Zugabe von a) 10 ml Wasser in 50 ml Tetrahydrofuran, b) 10 ml 2N Natronlauge und c) 10 ml Wasser in 50 ml Tetrahydrofuran und 100 g Natriumsulfat wird das Reaktionsgemisch aufgearbeitet. Die Suspension wird filtriert und das Filtrat eingeengt. Der Rückstand wird an Silicagel chromatographisch gereinigt.
DC (Silicagel, Toluol/Aethylacetat 1:1) Rf = 0,36;
IR ($CH_2Cl_2$): 3600, 1580, 1550, 1375 $cm^{-1}$.

B. 4-Chlor-2-hydroxymethyl-1-methyl-pyridiniumjodid

1,29 g (8,9 mMol) 4-Chlor-2-hydroxymethyl-pyridin werden in 5 ml Acetonitril gelöst, mit 10 ml Methyljodid versetzt und während 60 h bei Raumtemperatur stehen gelassen. Die entstandenen Kristalle werden abfiltriert und mit wenig Acetonitril und Aether gewaschen.
DC (Opti $UPC_{12}$, Wasser/Acetonitril 4:1) Rf = 0,38;
IR (KBr): 1632, 1570, 1496, 1430, 1400 $cm^{-1}$.

C. 4-Chlor-2-N,N-dimethylcarbamoyl-pyridin

Eine Lösung von 35 g (0,2 Mol) 4-Chlor-picolinsäurechlorid in 300 ml Toluol werden mit einem Ueberschuss Dimethylamin bei etwa 10° versetzt. Die breiige Mischung wird zur Trockne eingeengt und zwischen Aethylacetat und Natriumhydrogencarbonatlösung verteilt. Die organische Phase wird über Natriumsulfat getrocknet und das Lösungsmittel im Vakuum abedampft. Der Rückstand wird an Silicagel mit Toluol als Laufmittel chromatoghraphisch gereinigt.
DC (Silicagel, Toluol/Aethylacetat 1:1) Rf = 0,17; IR ($CH_2Cl_2$): 1640, 1575, 1550, 1410 $cm^{-1}$.

D. 4-Chlor-1-methyl-2-N,N-dimethylcarbamoyl-pyridiniumjodid

Eine Lösung von 1,4 g (7,5 mMol) 4-Chlor-2-N,N-dinethylcarbamoyl-pyridin in 9,5 ml (0,146 Mol) Methyljodid wird bei 50° während 24 h gerührt. Das entstandene kristalline Produkt wird durch Filtration isoliert.
DC (Opti $UPC_{12}$, Wasser/Acetonitril 1:1) Rf = 0,36;
IR (KBr): 1655, 1600, 1410 $cm^{-1}$.

E. 4-Chlor-2-chlormethyl-pyridiniumchlorid

Eine Lösung von 25,1 g (174 mMol) 4-Chlor-2-hydroxymethyl-pyridin in 300 ml Chloroform wird bei etwa 20° mit 24,6 ml (338 mMol) Thionylchlorid versetzt. Es ensteht zuerst eine Suspension, nach leichtem Erwärmen entweicht Schwefeldioxid und es entsteht eine klare Lösung. Das Reaktionsgemisch wird währen 45 min am Rückfluss erwärmt. Dann wird auf Raumtemperatur abgekühlt und mit 250 ml Toluol verdünnt. Due ausgefallenen Kristalle werden abfiltriert und getrocknet.

F. 2-Aminomethyl-4-chlor-pyridin, Dihydrochlorid

Eine Lösung von 9 g (45 mMol) 4-Chlor-2-chlormethyl-pyridiniumchlorid in 250 ml 25 %iger wässriger Ammoniaklösung und 30 ml Methanol werden 1,5 h am Rückfluss erwärmt. Anschliessend wird das Methanol im Vakuum abgedampft und das Produkt mit Aethylacetat extrahiert. Die Lösung wird mit einem Ueberschuss 1M Chlorwasserstoff in Methanol versetzt. wobei das Dihydrochlorid auskristallisiert.
DC (Silicagel, Methylenchlorid/Methanol 9:1) Rf = 0,25.

G. 2-Allyloxycarbonylaminomethyl-4-chlor-pyridin

6,8 g (38 mMol) 2-Aminomethyl-4-chlor-pyridin, Dihydrochlorid werden in 70 ml Wasser gelöst und der pH der Lösung mit 1N NaOH auf 9.5 eingestellt. Dann werden 6,7 ml (63 mMol) Chlorameisensäureallyl- ester zugetropft und während 1,5 h bei Raumtemperatur gerührt. Die Lösung wird mit Aethylacetat extrahiert, die organische Phase über Natriumsulfat getrocknet und eingeengt. Der Rückstand wird an Silicagel mit Toluol/Aethylacetat 4:1 chromatographisch gereinigt.
DC (Silicagel, Toluol/Aethylacetat 1:1) Rf = 0,66;
IR ($CH_2Cl_2$): 3440, 1720, 1580, 1510 $cm^{-1}$.

H. 2-Allyloxycarbonylaminomethyl-4-chlor-1-methyl-pyridiniumjodid

Eine Lösung von 4,5 g (19,8 mMol) 2-Allyloxycarbonylaminomethyl-4-chlor-pyridin in 41 ml Methyljodid wird während 3 Tagen bei Raumtemperatur stehen gelassen. Das Reaktionsgemisch wird eingeengt, der Rückstand in Methanol aufgenommen und durch Zugabe von Aethylacetat und Hexan zur Kristallisation gebracht.
DC (Opti $UPC_{12}$, Wasser/Acetonitril 1:1) Rf = 0,3.

I. 2-Chlor-5-hydroxymethyl-pyridin

Eine Lösung von 8 kg (46 mMol) 6-Chlor-nicotinsäuremethylester in 100 ml Tetrahydrofuran wird portionenweise bei etwa 5° mit 1,15 g (30,2 mMol) Lithiumaluminiumhydrid versetzt und während 3,5 h bei 10° gerührt. Durch Zugabe von a) 1,15 ml Wasser, b) 1,15 ml 2N Natronlauge und c) 1,15 ml Wasser und 10 g Natriumsulfat wird das Reaktionsgemisch aufgearbeitet. Die Suspension wird filtriert und das Filtrat eingeengt. Der Rückstand wird an Silicagel chromatographisch gereinigt.
DC (Silicagel, Toluol/Aethylacetat 1:1) Rf = 0,2.

J. 2-Chlor-5-hydroxymethyl-1-methyl-pyridiniumjodid

4 g (27,8 mMol) 2-Chlor-5-hydroxymethyl-pyridin werden in 20 ml Acetonitril gelöst, mit 37 ml Methyljodid versetzt und während 95 h bei Raumtemperatur gerührt. Die Suspension wird mit Aethylacetat verrührt, das Produkt abfiltriert und getrocknet.
DC (Opti $UPC_{12}$. Wasser/Acetonitril 7:3) Rf = 0,71;
IR (DMSO-$d_6$): 1622, 1509, 1434 $cm^{-1}$.

K. 6-Chlor-nicotinsäureamid

In eine Suspension von 25 g (0,145 Mol) 6-Chlor-nicotinsäuremethylester in 450 ml Aethylenglykol wird unter Zusatz von 0,5 g Ammoniumchlorid bei 20°-40° Ammoniakgas eingeleitet. Nach ca. 1,5 h werden die Kristalle abfiltriert und mit Aethanol gewaschen.
DC (Silicagel, Aethylacetat) Rf = 0,37;
IR (KBr): 1655, 1581, 1407 $cm^{-1}$.

L. 5-Carbamoyl-2-chlor-1-methyl-pyridiniumjodid

1 g (6,4 mMol) 6-Chlor-nicotinsäureamid werden in einem Gemisch von 6 ml Acetonitril und 6 ml Methyljodid gelöst und im Bombenrohr während 48 h auf 80° erwärmt. Das Reaktionsgemisch wird auf Raumtemperatur gebracht und mit Aethylacetat versetzt. Das kristalline Produkt wird abfiltriert und mit Aethylacetat gewaschen.
DC (Opti $UPC_{12}$, Wasser/Acetonitril 7:3) Rf = 0,5;
IR (KBr): 1684, 1628, 1558, 1497 $cm^{-1}$.

M. 2-Chlor-5-N-trichloracetylcarbamoyloxymethyl-pyridin

3,7 g (25,7 mMol) 2-Chlor-5-hydroxymethyl-pyridin werden in 150 ml Methylenchlorid gelöst und unter Stickstoff bei Raumtemperatur mit 3,1 ml (25,7 mMol) Trichloracetylisocyanat versetzt. Die weisse Suspension wird 1,5 h bei Raumtemperatur nachgerührt und das Produkt abfiltriert.
DC (Silicagel, Toluol/Aethylacetat 1:1) Rf = 0,56;
IR (DMSO-$d_6$): 1728, 1589, 1569, 1461 $cm^{-1}$.

N. 5-Carbamoyloxymethyl-2-chlor-pyridin

9,1 g (27,4 mMol) 2-Chlor-5-N-trichloracetylcarbamoyloxymethyl-pyridin werden in 1 l Methanol gelöst und mit 60 g Kieselgel bei Raumtemperatur während 24 h gerührt. Das Kieselgel wird abfiltriert, das Filtrat eingeengt und der Rückstand mit Hexan verrührt.
DC (Silicagel, Toluol/Aethylacetat 1:1) Rf = 0,24;
IR (DMSO-$d_6$): 1728, 1589, 1569, 1461 $cm^{-1}$.

O. 5-Carbamoyloxymethyl-2-chlor-1-methyl-pyridiniumjodid

1,65 g (8,84 mMol) 5-Carbamoyloxymethyl-2-chlor-pyridin werden in 20 ml Acetonitril und 10 ml Methyljodid während 22 h auf 60° erwärmt. Die Reaktionsmischung wird mit Aethylacetat verdünnt und das Produkt abfiltriert.
DC (Opti $UPC_{12}$, Wasser/Acetonitril 9:1) Rf = 0,38;
IR (KBr): 1718, 1700, 1591, 1500 $cm^{-1}$.

P. 2-Chlor-5-chlormethyl-pyridin

Die Titelverbindung wird in analoger Weise, wie unter E.) beschrieben, hergestellt.
DC (Silicagel, Toluol/Aethylacetat 1:1) Rf = 0,75;
IR ($CDCl_3$): 1558, 1565, 1462 $cm^{-1}$.

Q. 5-Azidomethyl-2-chlor-pyridin

5 g (30,8 mMol) 2-Chlor-5-chlormethyl-pyridin werden in 15 ml Dimethylformamid gelöst und mit 2,2 g (33,8 mMol) Natriumazid bei Raumtemperatur während 1 h gerührt. Die Reaktionslösung wird zwischen Wasser und Aethylacetat verteilt, die organische Phase mit Natriumsulfat getrocknet und das Lösungsmittel abgedampft. Dabei kristallisiert das Produkt aus.
DC (Silicagel, Toluol/Aethylacetat 4:1) Rf = 0,68;
IR ($CDCl_3$): 2104, 1589, 1568, 1463 $cm^{-1}$.

R. 5-Azidomethyl-2-chlor-1-methyl-pyridiniumjodid

4 g (23,7 mMol) 5-Azidomethyl-2-chlor-pyridin werden in 25 ml Methyljodid während 16 h auf 60° erwärmt. Das kristalline Produkt wird abfiltriert und mit Aethylacetat gewaschen.
DC (Opti $UPC_{12}$, Wasser/Acetonitril) Rf = 0,28;
IR (KBr): 2102, 1622, 1569, 1495 $cm^{-1}$.

Beispiel 1: (5R,6S)-6-[(1R)-1-Hydroxyäthyl]-2-[(1-methyl-4-pyridinio)-aminomethyl]-2-penem-3-carboxylat

600 mg (2,45 mMol) (5R,6S)-2-Aminomethyl-6-[(1R)-1-hydroxyäthyl]-2-penem-3-carbonsäure werden in 18,6 ml Wasser gelöst und der pH der Lösung bei 0° mit 1N Natronlauge auf 8,5 eingestellt. Nach der Zugabe von 759 mg (2,97 mMol) 4-Chlor-1-methyl-pyridiniumjodid wird die Reaktionslösung unter Einhalten des pH-Wertes auf 8,5 während 4,75 Stunden bei Raumtemperatur gerührt. Dann wird die Lösung mit Aethylacetat extrahiert und die wässerige Phase lyophilisiert. Der Rückstand wird an 70 g Opti $UPC_{12}$ (Laufmittel $H_2O$) chromatographisch gereinigt. Die Titelsubstanz wird durch Lyophilisation erhalten.
DC (Opti $UPC_{12}$; Wasser/Acetonitril 80:20) Rf = 0,45;
UV (Wasser) $\lambda_{max}$ = 282 nm;

IR (DMSO-$d_6$): 1770; 1650, 1625, 1540 $cm^{-1}$.

Beispiel 2: (5R,6S)-2-[(1-Benzyl-4-pyridinio)-aminomethyl]-6-[(1R)-1-hydroxyäthyl]-2-penem-3-carboxylat

Eine Lösung von 263 mg (1,08 mMol) (5R, 6S)-2-Aminomethyl-6-[(1R)-1-hydroxyäthyl]-2-penem-3-carbonsäure in 8,2 ml Wasser wird mit 1 ml 1N Natronlauge auf pH 8,5 eingestellt. Zu dieser Lösung werden bei Raumtemperatur 500 mg (ca. 1,2 mMol) 1-Benzyl-4-fluor-pyridiniumjodid (gelöst in 1,6 ml Wasser) zugetropft. Durch Versetzten mit 1N Natronlauge wird der pH der Lösung konstant bei 8,5 gehalten. Nach 75 Minuten Rühren wird der pH der Lösung auf 7 eingestellt. Das Reaktionsgemisch wird nach 2,5 Stunden am Rotationsverdampfer eingeengt und der Rückstand durch Chromatographie an 30 g Opti $UPC_{12}$ Kieselgel mit dem Laufmittelsystem Wasser/ Acetonitril 80:20 gereinigt. Durch Einengen der wässerigen Phase erhält man das amorphe Produkt.
DC (Opti $UPC_{12}$; Wasser/Acetonitril 70:30) Rf = 0,3;
UV (Wasser) $\lambda_{max}$ = 283 nm;
IR (DMSO-$d_6$): 1770; 1650, 1620, 1550 $cm^{-1}$.

Beispiel 3:
(5R,6S)-6-[(1R)-1-Hydroxyäthyl]-2-[(2-hydroxymethyl-1-methyl-4-pyridinio)-aminomethyl]-2-penem-3-carboxylat

3,6 g (14,7 mMol) (5R,6S)-2-Aminomethyl-6-[(1R)-1-hydroxyäthyl]-2-penem-3-carbonsäure werden in 100 ml Wasser gelöst und der pH der Lösung bei 0° mit 1N Natronlauge auf 7,7 gestellt. Nach der Zugabe von 5,05 g (17,7 mMol) 4-Chlor-2-hydroxymethyl-1-methyl-pyridiniumjodid wird die Reaktionslösung unter Einhaltung des pH-Wertes auf 7,5-7,9 während 5 h bei Raumtemperatur gerührt. Die Reaktionslösung wird lyophilisiert und der Rückstand an 200 g Opti $UPC_{12}$ (Laufmittel $H_2O$) chromatographisch gereinigt. Die Titelsubstanz wird durch Lyophilisation erhalten.
DC (Opti $UPC_{12}$, Wasser/Acetonitril 9:1) Rf = 0,42;
UV (Wasser) $\lambda_{max}$ = 280 nm;
IR (DMSO-$d_6$): 1773, 1649, 1618, 1350 $cm^{-1}$.

Beispiel 4: (5R,6S)-6-[(1R)-1-Hydroxyäthyl]-2-[(1-methyl-2-pyridinio)-aminomethyl]-2-penem-3-carboxylat

800 mg (3,28 mMol) (5R,6S)-2-Aminomethyl-6-[(1R)-1-hydroxyäthyl]-2-penem-3-carbonsäure werden in 15 ml Wasser gelöst und der pH der Lösung bei 0° mit 1N Natronlauge auf 8,0 eingestellt. Nach der Zugabe von 1,02 g (4,0 mMol) 2-Chlor-1-methyl-pyridiniumjodid wird die Reaktionslösung unter Einhalten des pH-Wertes auf 8,5 während 3 h bei Raumtemperatur gerührt. Dann wird die wässerige Phase lyophilisiert. Der Rückstand wird an 70 g Opti $UPC_{12}$ (Laufmittel $H_2O$) chromatographisch gereinigt. Die Titelsubstanz wird durch Lyophilisation erhalten.
DC (Opti $UPC_{12}$, Wasser/Acetonitril 9:1) Rf = 0,38;
UV (Wasser) $\lambda_{max}$ = 312 nm;
IR (DMSO-$d_6$): 1773, 1648, 1620, 1590, 1542 $cm^{-1}$.

Beispiel 5: (5R,6S)-6-[(1R)-1-Hydroxyäthyl]-2-[2-(1-methyl-2-pyridinioamino)-äthyl]-2-penem-3-carboxylat

300 mg (1,16 mMol) (5R,6S)-2-(2-Aminoäthyl)-6-[(1R)-1-hydroxyäthyl]-2-penem-3-carbonsäure werden in 20 ml Wasser gelöst und der pH der Lösung bei 0° mit 0,1N Natronlauge auf 8,0 eingestellt. Nach der Zugabe von 534 mg (2,1 mMol) 2-Chlor-1-methyl-pyridiniumjodid wird die Reaktionslösung unter Einhalten des pH-Wertes auf 8,5-9,0 während 23 h bei Raumtemperatur gerührt. Dann wird die wässerige Phase lyophilisiert. Der Rückstand wird an 70 g Opti $UPC_{12}$ (Laufmittel $H_2O$) chromatographisch gereinigt. Die Titelsubstanz wird durch Lyophilisation erhalten.
DC (Opti $UPC_{12}$, Wasser/Acetonitril 9:1) Rf = 0,22;
UV (Wasser) $\lambda_{max}$ = 307 nm;
IR (DMSO-$d_6$): 1772, 1650, 1625, 1581, 1543 $cm^{-1}$.

Beispiel 6:
(5R,6S)-6-[(1R)-1-Hydroxyäthyl]-2-[(2-carboxy-1-methyl-4-pyridinio)-aminomethyl]-2-penem-3-carboxylat, Natrimsalz

1,5 g (6,14 mMol) (5R,6S)-2-Aminomethyl-6-[(1R)-1-hydroxyäthyl]-2-penem-3-carbonsäure werden in 20 ml Wasser gelöst und der pH der Lösung bei 0° mit 0,1N Natronlauge auf 7,5 eingestellt. Nach der Zugabe von 13,2 mg (32,6 mMol) 4-Chlor-1-methyl-2-methoxycarbonyl-pyridiniumjodid wird die Reaktionslösung unter Einhalten des pH-Wertes auf 7,5 während 16 h bei Raumtemperatur gerührt. Dann wird die wässerige Phase lyophilisiert. Der Rückstand wird an 70 g Opti $UPC_{12}$ (Laufmittel $H_2O$) chromatographisch gereinigt. Die Titelsubstanz wird durch Lyophilisation erhalten.
DC (Opti $UPC_{12}$, Wasser/Acetonitril 9:1) Rf = 0,34;
UV (Wasser) $\lambda_{max}$ = 282 nm;
IR (DMSO-$d_6$): 1775, 1637, 1347 $cm^{-1}$.

Beispiel 7:
(5R,6S)-6-[(1R)-1-Hydroxyäthyl]-2-[(2-allyloxycarbonylaminomethyl-1-methyl-4-pyridinio)-aminomethyl]-2-penem-3-carboxylat

1,5 g (6,1 mMol) (5R,6S)-2-Aminomethyl-6-[(1R)-1-hydroxyäthyl]-2-penem-3-carbonsäure werden in 30 ml Wasser gelöst und der pH der Lösung bei 0° mit 0,1N Natronlauge auf 8,5 eingestellt. Nach der Zugabe von 3,2 g (8,6 mMol) 2-Allyloxycarbonylaminomethyl-4-chlor-1-methyl-pyridiniumjodid wird die Reaktionslösung unter Einhalten des pH-Wertes auf 8,5 während 6,5 h bei Raumtemperatur gerührt. Dann wird die wässerige Phase lyophilisiert. Der Rückstand wird an 70 g $XAD_2$ (Laufmittel $H_2O$) chromatographisch gereinigt. Die Titelsubstanz wird durch Lyophilisation erhalten.
DC (Opti $UPC_{12}$, Wasser/Acetonitril 7:3) Rf = 0,3;
UV (Wasser) $\lambda_{max}$ = 280 nm;
IR (DMSO-$d_6$): 1772, 1719, 1649, 1619 $cm^{-1}$.

Beispiel 8:
(5R,6S)-6-[(1R)-1-Hydroxyäthyl]-2-[(2-aminomethyl-1-methyl-4-pyridinio)-aminomethyl]-2-penem-3-carboxylat

1,4 g (3,1 mMol) (5R,6S)-6-[(1R)-1-Hydroxyäthyl]-2-[(2-allyloxycarbonylaminomethyl-1-methyl-4-pyridinio)-2-penem-3-carboxylat werden in 50 ml Dimethylformamid gelöst und mit 0,54 g (3,45 mMol) Dimethylbarbitursäure versetzt. Anschliessend werden unter Stickstoff 300 mg Tetrakis-(triphenylphosphin)-palladium zugegeben. Es wird 4 h bei Raumtemperatur gerührt. Das Lösungsmittel wird am Hochvakuum abgedampft und der Rückstand zwischen gesättigter Natriumhydrogencarbonatlösung und Aethylacetat verteilt. Die wässerige Phase wird lyophilisiert und der Rückstand an 80 g Opti $UPC_{12}$ mit Wasser chromatographiert. Die Titelsubstanz wird durch Lyophilisation erhalten.
DC (Opti $UPC_{12}$, Wasser/Acetonitril) Rf = 0,25;
UV (Wasser) $\lambda_{max}$ = 280 nm;
IR (DMSO-$d_6$): 1773, 1649, 1618 $cm^{-1}$.

Beispiel 9:
(5R.6S)-6-[(1R)-1-Hydroxyäthyl}-2-[(5-azidomethyl-1-methyl-2-pyridinio)-aminomethyl]-2-penem-3-carboxylat

In analoger Weise, wie in den Beispielen 1-8 beschrieben, wird (5R,6S)-2-Aminomethyl-6-[(1R)-1-hydroxyäthyl]-2-penem-3-carbonsäure mit 5-Azidomethyl-2-chlor-1-methyl-pyridiniumjodid zur Titelverbindung umgesetzt.
DC (Opti $UPC_{12}$, Wasser/Acetonitril 9:1) Rf = 0,43;
UV (Wasser) $\lambda_{max}$ = 315 nm;
IR (DMSO-$d_6$): 2101, 1773, 1659, 1619, 1576 $cm^{-1}$.

Beispiel 10:
(5R,6S)-6-[(1R)-1-Hydroxyäthyl]-2-[(5-aminomethyl-1-methyl-2-pyridinio)-aminomethyl]-2-penem-3-carboxylat

279 mg (0.71 mMol) (5R,6S)-6-[(1R)-1-Hydroxyäthyl]-2-[(5-azidomethyl-1-methyl-2-pyridinio)-aminomethyl]-2-penem-3-carboxylat, gelöst in 60 ml Wasser, werden nach Zugabe von 150 mg Pd/C 10% mit Wasserstoff unter Normaldruck und bei Raumtemperatur während 2 h hydriert. Anschliessend wird der Katalysator abfiltriert und das Produkt durch Lyophilisation isoliert.
DC (Opti $UPC_{12}$, Wasser/Acetonitril 9:1) Rf = 0,39;
UV (Wasser). $\lambda_{max}$ = 314 nm;
IR (DMSO-$d_6$): 1773, 1592 $cm^{-1}$.

Beispiel 11: In analoger Weise, wie in den Beispielen 1-10 beschrieben, erhält man die folgenden Verbindungen:

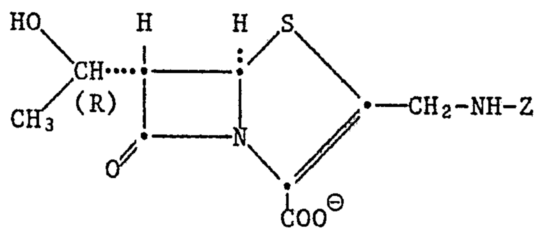

Z

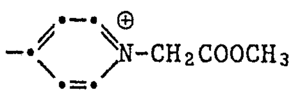

DC (Opti $UPC_{12}$; Wasser/Acetonitril 80:20)
Rf = 0,40;
UV (Wasser) $\lambda_{max}$ = 282 nm;
IR (DMSO-$d_6$): 1770, 1735, 1650, 1620 $cm^{-1}$.

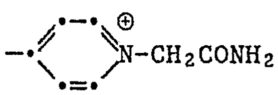

DC (Opti $UPC_{12}$; Wasser/Acetonitril 80:20)
Rf = 0,55;
UV (Wasser) $\lambda_{max}$ = 283 nm;
IR (DMSO-$d_6$): 1775, 1710, 1650, 1625 $cm^{-1}$.

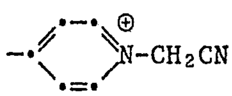

DC (Opti $UPC_{12}$; Wasser/Acetonitril 70:30)
Rf = 0,40;
UV (Wasser) $\lambda_{max}$ = 283 nm;
IR (DMSO-$d_6$): 1770, 1650, 1625 $cm^{-1}$.

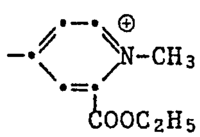

DC (Opti $UPC_{12}$; Wasser/Acetonitril 80:20)
Rf = 0,55;
UV (Wasser) $\lambda_{max}$ = 283 nm;
IR (DMSO-$d_6$): 1770, 1730, 1650, 1625 $cm^{-1}$.

Z

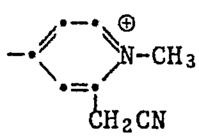

DC (Opti $UPC_{12}$; Wasser/Acetonitril 80:20)
Rf = 0,55;
UV (Wasser) $\lambda_{max}$ = 283 nm;
IR (DMSO-$d_6$): 1775, 1650, 1625 $cm^{-1}$.

Z

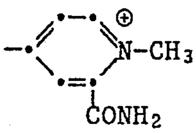

DC (Opti $UPC_{12}$; Wasser)
Rf = 0,31;
UV (Wasser) $\lambda_{max}$ = 285 nm;
IR (DMSO-$d_6$): 1773, 1696, 1644 $cm^{-1}$.

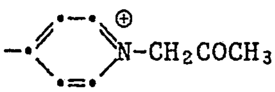

DC (Opti $UPC_{12}$; Wasser/Acetonitril 1:1)
Rf = 0,50;
UV (Wasser) $\lambda_{max}$ = 283 nm;
IR (DMSO-$d_6$): 1773, 1733, 1652, 1618 $cm^{-1}$.

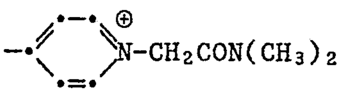

DC (Opti $UPC_{12}$; Wasser/Acetonitril 1:1)
Rf = 0,50;
UV (Wasser) $\lambda_{max}$ = 282 nm;
IR (DMSO-$d_6$): 1772, 1664, 1618 $cm^{-1}$.

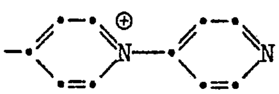

DC (Opti $UPC_{12}$; Wasser/Acetonitril 1:1)
Rf = 0,53;
UV (Wasser) $\lambda_{max}$ = 300 nm;
IR (DMSO-$d_6$): 1773, 1651, 1620, 1584 $cm^{-1}$.

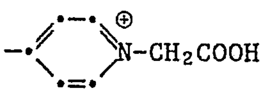

DC (Opti $UPC_{12}$; Wasser/Acetonitril 9:1)
Rf = 0,45;
UV (Wasser) $\lambda_{max}$ = 283 nm;
IR (DMSO-$d_6$): 1773, 1651 $cm^{-1}$.

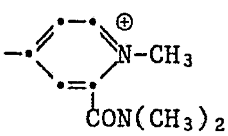

DC (Opti $UPC_{12}$; Wasser/Acetonitril 9:1)
Rf = 0,26;
UV (Wasser) $\lambda_{max}$ = 283 nm;
IR (DMSO-$d_6$): 1772, 1643, 1619, 1406 $cm^{-1}$.

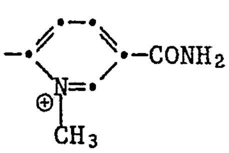

DC (Opti $UPC_{12}$; Wasser/Acetonitril 9:1)
Rf = 0,37;
UV (Wasser) $\lambda_{max}$ = 313 nm;
IR (DMSO-$d_6$): 1774, 1687, 1651, 1621 $cm^{-1}$.

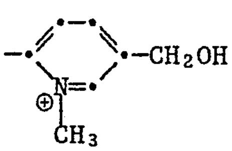

DC (Opti $UPC_{12}$; Wasser/Acetonitril 7:3)
Rf = 0,68;
UV (Wasser) $\lambda_{max}$ = 314 nm;
IR (DMSO-$d_6$): 1772, 1658, 1619, 1594 $cm^{-1}$.

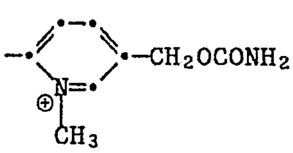

DC (Opti $UPC_{12}$; Wasser/Acetonitril 9:1)
Rf = 0,36;
UV (Wasser) $\lambda_{max}$ = 313 nm;
IR (DMSO-$d_6$): 1773, 1726, 1661, 1594 $cm^{-1}$.

Z

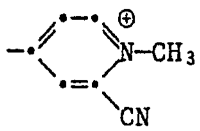

DC (Opti $UPC_{12}$; Wasser/Acetonitril 9:1)
Rf = 0,30;
UV (Wasser) $\lambda_{max}$ = 286 nm;
IR (DMSO-$d_6$): 2120, 1773, 1642, 1622 $cm^{-1}$

Beispiel 12: In analoger Weise, wie in den vorangehenden Beispielen beschrieben, werden die folgenden Verbindungen hergestellt:

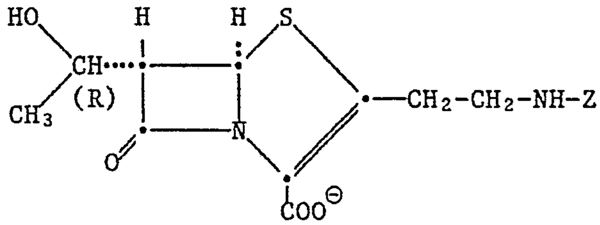

Z

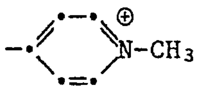

DC (Opti $UPC_{12}$; Wasser/Acetonitril 80:20)
Rf = 0,50;
UV (Wasser) $\lambda_{max}$ = 282 nm;
IR (DMSO-$d_6$): 1770, 1650, 1625 $cm^{-1}$.

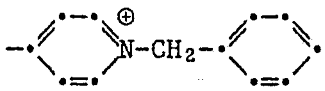

DC (Opti $UPC_{12}$; Wasser/Acetonitril 70:30)
Rf = 0,35;
UV (Wasser) $\lambda_{max}$ = 283 nm;
IR (DMSO-$d_6$): 1770, 1650, 1620 $cm^{-1}$.

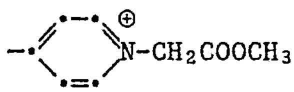

DC (Opti $UPC_{12}$; Wasser/Acetonitril 80:20)
Rf = 0,45;
UV (Wasser) $\lambda_{max}$ = 282 nm;
IR (DMSO-$d_6$): 1770, 1735, 1650, 1620 $cm^{-1}$.

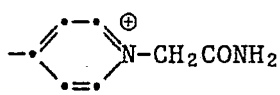

DC (Opti $UPC_{12}$; Wasser/Acetonitril 80:20)
Rf = 0,60;
UV (Wasser) $\lambda_{max}$ = 283 nm;
IR (DMSO-$d_6$): 1775, 1710, 1650, 1625 $cm^{-1}$.

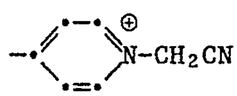

DC (Opti $UPC_{12}$; Wasser/Acetonitril 70:30)
Rf = 0,95;
UV (Wasser) $\lambda_{max}$ = 283 nm;
IR (DMSO-$d_6$): 1770, 1650, 1625 $cm^{-1}$.

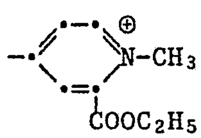

DC (Opti $UPC_{12}$; Wasser/Acetonitril 80:20)
Rf = 0,65;
UV (Wasser) $\lambda_{max}$ = 283 nm;
IR (DMSO-$d_6$): 1770, 1730, 1650, 1625 $cm^{-1}$.

Z

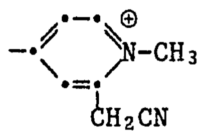

DC (Opti $UPC_{12}$; Wasser/Acetonitril 80:20)
Rf = 0,60;
UV (Wasser) $\lambda_{max}$ = 283 nm;
IR ($DMSO-d_6$): 1775, 1650, 1625 $cm^{-1}$.

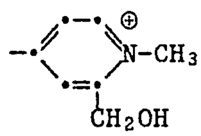

DC (Opti $UPC_{12}$; Wasser/Acetonitril 9:1)
Rf = 0,26;
IR ($DMSO-d_6$): 1771, 1651, 1619 $cm^{-1}$.

Beispiel 13: Trockenampullen oder Vials, enthaltend 0,5 g (5R, 6S)-6-[(1R)-1-Hydroxyäthyl]-2-[(2-hydroxymethyl-1-methyl-4-pyridinio)-aminomethyl]-2-penem-3-carboxylat als Wirksubstanz, werden wie folgt hergestellt:

Zusammensetzung (für 1 Ampulle oder Vial):

Wirksubstanz 0,5 g
Mannit 0,5 g

Eine sterile wässerige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorgangehenden Beispiele verwendet werden.

**Patentansprüche**

1. Verbindungen der Formel

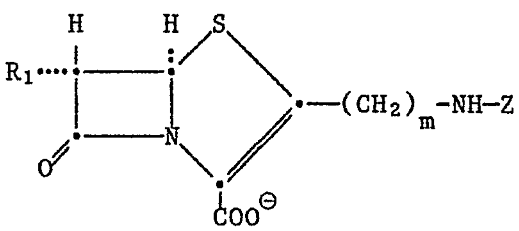

(I),

worin $R_1$ für Hydroxymethyl oder 1-Hydroxyäthyl steht, Z für einen Rest

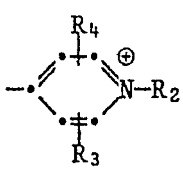

oder

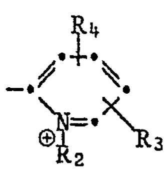

steht, worin $R_2$ gegebenenfalls substituiertes Niederalkyl, Niederalkenyl, gegebenenfalls substituiertes Phenyl, Pyridyl oder veräthertes Hydroxy bedeutet, $R_3$ und $R_4$ unabhängig voneinander für Wasserstoff, gegebenenfalls substituiertes Niederalkyl, gegebenenfalls funktionell abgewandeltes Carboxyl, veräthertes Hydroxy, verestertes Hydroxy oder gegebenenfalls substituiertes Amino stehen und m eine ganze Zahl von 1 bis 4 ist, und Salze von Verbindungen der Formel I.

2. Verbindungen der Formel

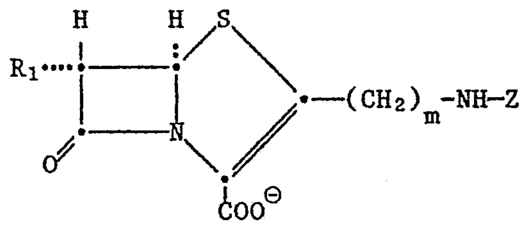

(I),

worin $R_1$ für Hydroxymethyl oder 1-Hydroxyäthyl steht, Z für den Rest

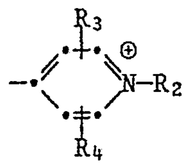

steht, worin $R_2$ gegebenenfalls substituiertes Niederalkyl, Niederalkenyl, gegebenenfalls substituiertes Phenyl oder veräthertes Hydroxy bedeutet, $R_3$ und $R_4$ unabhängig voneinander für Wasserstoff, gegebenenfalls substituiertes Niederalkyl, gegebenenfalls funktionell abgewandeltes Carboxyl, veräthertes Hydroxy, Halogen oder gegebenenfalls substituiertes Amino stehen und m eine ganze Zahl von 1 bis 4 ist, und Salze von Verbindungen der Formel I.

3. Verbindungen der Formel I gemäss Anspruch 1, worin $R_1$ Hydroxymethyl oder (1R)-1-Hydroxyäthyl ist, Z für einen Rest

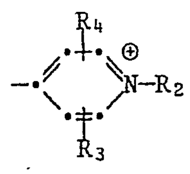

oder

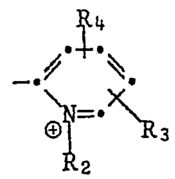

steht, worin $R_2$ Niederalkyl, durch Hydroxy, Halogen, Niederalkanoyloxy, Carbamoyloxy, Niederalkoxy, Carboxyl, Niederalkoxycarbonyl, Carbamoyl, N-Niederalkyl- oder N,N-Diniederalkylcarbamoyl, Cyano, Amino, Azido, Phenyl, durch Niederalkyl, Niederalkoxy, Halogen, Cyano und/oder Carboxy substituiertes Phenyl oder Oxo substituiertes Niederalkyl; Niederalkenyl, Phenyl, durch Niederalkyl, Niederalkoxy, Halogen, Cyano und/oder Carboxy substituiertes Phenyl, Pyridyl oder Niederalkoxy bedeutet, $R_3$ und $R_4$ unabhängig voneinander für Wasserstoff, Niederalkyl, durch Hydroxy, Halogen, Niederalkanoyloxy, Carbamoyloxy, Niederalkoxy, Carboxy, Niederalkoxycarbonyl, Carbamoyl, N-Niederalkyl- oder N,N-Diniederalkylcarbamoyl, Cyano, Amino, Azido, Phenyl, durch Niederalkyl, Niederalkoxy, Halogen, Cyano und/oder Carboxy substituiertes Phenyl oder Oxo substituiertes Niederalkyl; Carboxyl, Niederalkoxycarbonyl, Carbamoyl, N-Niederalkyl- oder N,N-Diniederalkylcarbamoyl, Cyano, Niederalkoxy, Halogen, Niederalkanoyloxy, Carbamoyloxy, Amino, Niederalkylamino, Diniederalkylamino, Niederalkylenamino oder Niederalkanoylamino stehen, m eine ganze Zahl von 1 bis 4 ist, und Salze von Verbindungen der Formel I.

4. Verbindungen der Formel I gemäss Anspruch 2, worin $R_1$ Hydroxymethyl oder (1R)-1-Hydroxyäthyl ist, $R_2$ Niederalkyl, durch Halogen, Niederalkoxy, Carboxyl, Niederalkoxycarbonyl, Carbamoyl, Cyano, Phenyl, durch Niederalkyl, Niederalkoxy, Halogen, Cyano und/oder Carboxy substituiertes Phenyl oder Oxo substituiertes Niederalkyl; Niederalkenyl, Phenyl, durch Niederalkyl, Niederalkoxy, Halogen, Cyano und/oder Carboxy substituiertes Phenyl, oder Niederalkoxy bedeutet, $R_3$ und $R_4$ unabhängig voneinander für Wasserstoff, Niederalkyl, durch Halogen, Niederalkoxy, Carboxy, Niederalkoxycarbonyl, Carbamoyl, Cyano, Phenyl, durch Niederalkyl, Niederalkoxy, Halogen, Cyano und/oder Carboxy substituiertes Phenyl oder Oxo substituiertes Niederalkyl; Carboxyl, Niederalkoxycarbonyl, Carbamoyl, Cyano, Niederalkoxy, Halogen, Amino, Niederalkylamino, Diniederalkylamino, Niederalkylenamino oder Niederalkanoylamino stehen, m eine ganze Zahl von 1 bis 4 ist, und Salze von Verbindungen der Formel I.

5. Verbindungen der Formel I gemäss Anspruch 1, worin $R_1$ (1R)-1-Hydroxyäthyl ist, Z für einen Rest

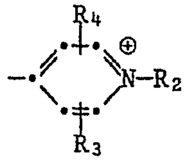

steht, worin $R_2$ Niederalkyl, Acetonyl oder durch Carboxy, Niederalkoxycarbonyl, Carbamoyl, N,N-Diniederalkylcarbamoyl oder Phenyl substituiertes Niederalkyl bedeutet, $R_3$ Wasserstoff bedeutet, $R_4$ für Wasserstoff, durch Hydroxy, Carboxy, Cyano oder Amino substituiertes Niederalkyl; carboxy, Carbamoyl oder Cyano steht, und m 1 oder 2 ist, und Salze von Verbindungen der Formel I.

6. Verbindungen der Formel I gemäss Anspruch 2, worin $R_1$ Hydroxymethyl oder (1R)-1-Hydroxyäthyl ist, $R_2$ Niederalkyl oder durch Carboxy, Niederalkoxycarbonyl, Carbamoyl, Cyano oder Phenyl substituiertes Niederalkyl bedeutet, $R_3$ Wasserstoff bedeutet, $R_4$ für Wasserstoff, durch Carboxy, Niederalkoxycarbonyl oder Cyano substituiertes Niederalkyl; Carboxy, Niederalkoxycarbonyl oder Cyano steht, und m 1 oder 2 ist, und Salze von Verbindungen der Formel I.

7. Verbindungen der Formel I gemäss Anspruch 1, worin $R_1$ (1R)-1-Hydroxyäthyl ist, Z für einen Rest

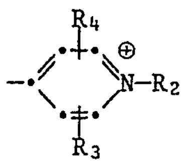

steht, worin $R_2$ Niederalkyl oder durch Carbamoyl substituiertes Niederalkyl ist, $R_3$ Wasserstoff ist und $R_4$ Wasserstoff oder durch Hydroxy substituiertes Niederalkyl bedeutet und m 1 ist, und Salze von Verbindungen der Formel I.

8. Verbindungen der Formel I gemäss Anspruch 2, worin $R_1$ (1R)-1-Hydroxyäthyl ist, $R_2$ Niederalkyl oder Phenylniederalkyl ist, $R_3$ und $R_4$ Wasserstoff bedeuten und m 1 ist, und Salze von Verbindungen der Formel I.

9. Pharmazeutisch annehmbare Salze von Verbindungen der Formel I gemäss einem der Ansprüche 1 oder 2.

10. (5R,6S)-6-[(1R)-1-Hydroxyäthyl]-2-[(1-methyl-4-pyridinio)-aminomethyl]-2-penem-3-carboxylat gemäss Anspruch 2.

11. (5R,6S)-2-[(1-Benzyl-4-pyridinio)-aminomethyl]-6-[(1R)-1-hydroxyäthyl]-2-penem-3-carboxylat gemäss Anspruch 2.

12. (5R,6S)-2-[(1-Aminocarbonylmethyl-4-pyridinio)-aminomethyl]-6-[(1R)-1-hydroxyäthyl]-2-penem-3-carboxylat gemäss Anspruch 2.

13. (5R,6S)-6-[(1R)-1-Hydroxyäthyl]-2-[(2-hydroxymethyl-1-methyl-4-pyridinio)-aminomethyl]-2-penem-3-carboxylat gemäss Anspruch 1.

14. (5R,6S)-6-[(1R)-1-Hydroxyäthyl]-2-[(2-carboxy-1-methyl-4-pyridinio)-aminomethyl]-2-penem-3-carboxylat und pharmazeutisch annehmbare Salze davon gemäss Anspruch 1.

15. (5R,6S)-6-[(1R)-1-Hydroxyäthyl]-2-[(2-aminomethyl-1-methyl-4-pyridinio)-aminomethyl]-2-penem-3-carboxylat und pharmazeutisch annehmbare Salze davon gemäss Anspruch 1.

16. (5R,6S)-6-[(1R)-1-Hydroxyäthyl]-2-[(1-acetonyl-4-pyridinio)-aminomethyl]-2-penem-3-carboxylat gemäss Anspruch 1.

17. Pharmazeutisches Präparat enthaltend eine Verbindung der Formel I gemäss Anspruch 1 oder ein pharmazeutisch annehmbares Salz davon.

18. Pharmazeutisches Präparat enthaltend eine Verbindung der Formel I gemäss Anspruch 2 oder ein pharmazeutisch annehmbares Salz davon.

19. Verwendung von Verbindungen der Formel I gemäss Anspruch 1 oder daren pharmazeutisch annehmbarer Salze sur Herstellung von pharmazeutischen Präparaten.

20. Verwendung von Verbindungen der Formel I gemäss Anspruch 2 oder deren pharmazeutisch annehmbarer Salze sur Herstellung von pharmazeutischen Präparaten.

21. Eine Verbindung der Formel I gemäss Anspruch 1 oder ein pharmazeutisch annehmbares Salz einer solchen Verbindungen zur Anwendung in einem Verfahren zur therapeutischen Behandlung des menschlichen oder tierischen Körpers.

22. Eine Verbindung der Formel I gemäss Anspruch 2 oder ein pharmazeutisch annehmbares Salz einer solchen Verbindung zur Anwendung in einem Verfahren zur therapeutischen Behandlung des menschlichen oder tierischen Körpers.

23. Verfahren zur Herstellung von Verbindungen der Formel I gemäss Anspruch 1, dadurch gekennzeichnet, dass man eine Penem-Verbindung der Formel

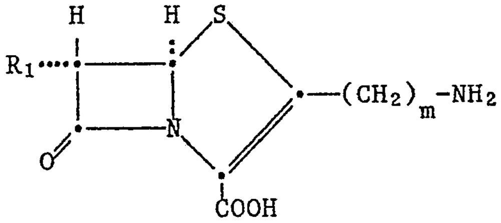

(II),

worin $R_1$ und m die unter Formel I angegebenen Bedeutungen haben, mit einer Pyridinium-Verbindung der Formel

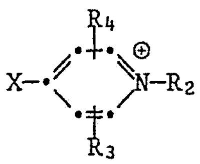 $Y^{\ominus}$ (III) oder 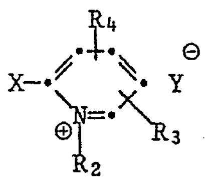 (IIIa),

worin $R_2$, $R_3$ und $R_4$ die unter Formel I angegebenen Bedeutungen haben, wobei funktionelle Gruppen in diesen Resten gegebenenfalls in geschützter Form vorliegen, X eine Abgangsgruppe ist und $Y^{\ominus}$ für ein Anion steht, umsetzt und, falls erforderlich, geschützte funktionelle Gruppen in den Resten $R_2$, $R_3$ und/oder $R_4$ in die freien funktionellen Gruppen überführt, und, wenn erwünscht, eine erhältliche freie Verbindung der Formel I in ein Salz überführt.

24. Verfahren zur Herstellung von Verbindungen der Formel I gemäss Anspruch 2, dadurch gekennzeichnet, dass man eine Penem-Verbindung der Formel

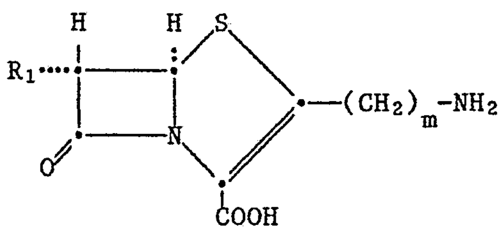

(II),

worin $R_1$ und m die unter Formel I angegebenen Bedeutungen haben, mit einer Pyridinium-Verbindung der Formel

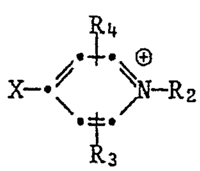

$Y^{\ominus}$ (III),

worin $R_2$, $R_3$ und $R_4$ die unter Formel I angegebenen Bedeutungen haben, wobei funktionelle Gruppen in diesen Resten gegebenenfalls in geschützter Form vorliegen, X eine Abgangsgruppe ist und $Y^{\ominus}$ für ein Anion steht, umsetzt und, falls erforderlich, geschützte funktionelle Gruppen in den Resten $R_2$, $R_3$ und/oder $R_4$ in die freien funktionellen Gruppen überführt, und, wenn erwünscht, eine erhältliche freie Verbindung der Formel I in ein Salz überführt.

25. Die nach dem Verfahren gemäss Anspruch 23 erhältlichen Verbindungen.

26. Die nach dem Verfahren gemäss Anspruch 24 erhältlichen Verbindungen.

Patentansprüche für die folgenden Vertragsstaaten AT, ES, GR

1. Verfahren zur Herstellung von Verbindung der Formel

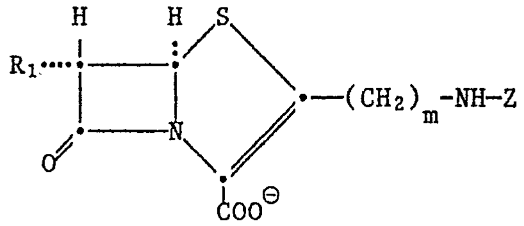

(I),

worin $R_1$ für Hydroxymethyl oder 1-Hydroxyäthyl steht, Z für einen Rest

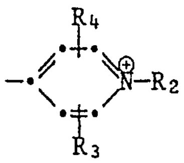

oder

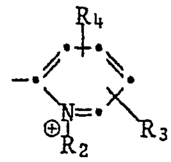

steht, worin $R_2$ gegebenenfalls substituiertes Niederalkyl, Niederalkenyl, gegebenenfalls substituiertes Phenyl, Pyridyl oder veräthertes Hydroxy bedeutet, $R_3$ und $R_4$ unabhängig voneinander für Wasserstoff, gegebenenfalls substituiertes Niederalkyl, gegebenenfalls funktionell abgewandeltes Carboxyl, veräthertes Hydroxy, verestertes Hydroxy oder gegebenenfalls substituiertes Amino stehen und m eine ganze Zahl von 1 bis 4 ist, und Salzen davon, dadurch gekennzeichnet, dass man eine Penem-Verbindung der Formel

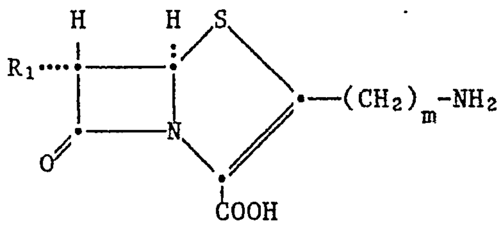

(II),

worin $R_1$ und m die unter Formel I angegebenen Bedeutungen haben, mit einer Pyridinium-Verbindung der Formel

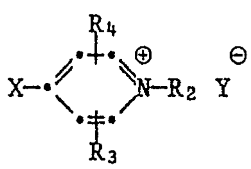

(III) oder

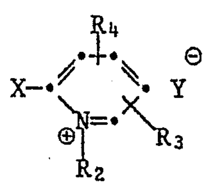

(IIIa),

worin $R_2$, $R_3$ und $R_4$ die unter Formel I angegebenen Bedeutungen haben, wobei funktionelle Gruppen in diesen Resten gegebenenfalls in geschützter Form vorliegen, X eine Abgangsgruppe ist und $Y^{\ominus}$ für ein Anion steht, umsetzt und, falls erforderlich, geschützte funktionelle Gruppen in den Resten $R_2$, $R_3$ und/oder $R_4$ in die freien funktionellen Gruppen überführt, und, wenn erwünscht, eine erhältliche freie Verbindung der Formel I in ein Salz überführt.

2. Verfahren zur Herstellung von Verbindungen der Formel

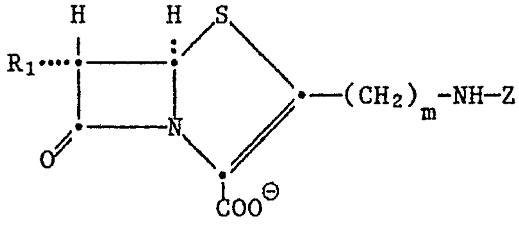

(I),

worin $R_1$ für Hydroxymethyl oder 1-Hydroxyäthyl steht, Z für den Rest

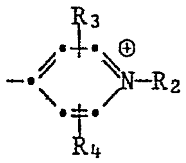

steht, worin $R_2$ gegebenenfalls substituiertes Niederalkyl, Niederalkenyl, gegebenenfalls substituiertes Phenyl oder veäthertes Hydroxy bedeutet, $R_3$ und $R_4$ unabhängig voneinander für Wasserstoff, gegebenenfalls substituiertes Niederalkyl, gegebenenfalls funktionell abgewandeltes Carboxyl, veräthertes Hydroxy, Halogen oder gegebenenfalls substituiertes Amino stehen und m eine ganze Zahl von 1 bis 4 ist, und Salzen davon, dadurch gekennzeichnet, dass man eine Penem-Verbindung der Formel

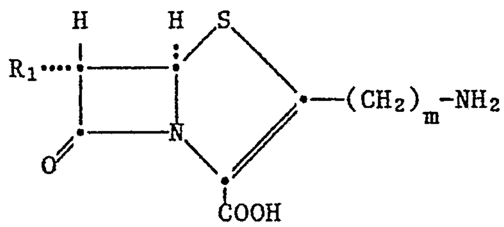 (II),

worin $R_1$ und m die unter Formel I angegebenen Bedeutungen haben, mit einer Pyridinium-Verbindung der Formel

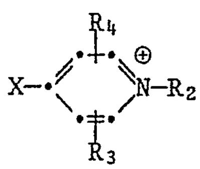 $Y^{\ominus}$ (III),

worin $R_2$, $R_3$ und $R_4$ die unter Formel I angegebenen Bedeutungen haben, wobei funktionelle Gruppen in diesen Resten gegebenenfalls in geschützter Form vorliegen, X eine Abgangsgruppe ist und $Y^{\ominus}$ für ein Anion steht, umsetzt und, falls erforderlich, geschützte funktionelle Gruppen in den Resten $R_2$, $R_3$ und/oder $R_4$ in die freien funktionellen Gruppen überführt, und, wenn erwünscht, eine erhältliche freie Verbindung der Formel I in ein Salz überführt.

3. Verfahren zur Herstellung von Verbindung der Formel I gemäss Anspruch 1, worin $R_1$ Hydroxymethyl oder (1R)-1-Hydroxyäthyl ist, Z für einen Rest

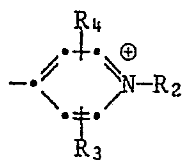 oder 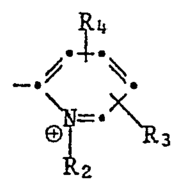

steht, worin $R_2$ Niederalkyl, durch Hydroxy, Halogen, Niederalkanoyloxy, Carbamoyloxy, Niederalkoxy, Carboxyl, Niederalkoxycarbonyl, Carbamoyl, N-Niederalkyl- oder N,N-Diniederalkylcarbamoyl, Cyano, Amino, Azido, Phenyl, durch Niederalkyl, Niederalkoxy, Halogen, Cyano und/oder Carboxy substituiertes Phenyl oder Oxo substituiertes Niederalkyl; Niederalkenyl, Phenyl, durch Niederalkyl, Niederalkoxy, Halogen, Cyano und/oder Carboxy substituiertes Phenyl, Pyridyl, oder Niederalkoxy bedeutet, $R_3$ und $R_4$ unabhängig voneinander für Wasserstoff, Niederalkyl, durch Hydroxy, Halogen, Niederalkanoyloxy, Carbamoyloxy, Niederalkoxy, Carboxy, Niederalkoxycarbonyl, Carbamoyl, N-Niederalkyl- oder N,N-Diniederalkylcarbomyl, Cyano, Amino, Azido, Phenyl, durch Niederalkyl, Niederalkoxy, Halogen, Cyano und/oder Carboxy substituiertes Phenyl oder Oxo substituiertes Niederalkyl; Carboxyl, Niederalkoxycarbonyl, Carbamoyl, N-Niederalkyl- oder N,N-Diniederalkylcarbamoyl, Cyano, Niederalkoxy, Halogen, Niederalkanoyloxy, Carbamoyloxy, Amino, Niederalkylamino, Diniederalkylamino, Niederalkylenamino oder Niederalkanoylamino stehen, m eine ganze Zahl von 1 bis 4 ist, und Salzen von Verbindungen der Formel I.

4. Verfahren zur Herstellung von Verbindung der Formel I gemäss Anspruch 2, worin $R_1$ Hydroxymethyl

oder (1R)-1-Hydroxyäthyl ist, $R_2$ Niederalkyl, durch Halogen, Niederalkoxy, Carboxyl, Niederalkoxycarbonyl, Carbamoyl, Cyano, Phenyl, durch Niederalkyl, Niederalkoxy, Halogen, Cyano und/oder Carboxy substituiertes Phenyl oder Oxo substituiertes Niederalkyl; Niederalkenyl Phenyl, durch Niederalkyl, Niederalkoxy, Halogen, Cyano und/oder Carboxy substituiertes Phenyl, oder Niederalkoxy bedeutet, $R_3$ und $R_4$ unabhängig voneinander für Wasserstoff, Niederalkyl, durch Halogen, Niederalkoxy, Carboxy, Niederalkoxycarbonyl, Carbamoyl, Cyano, Phenyl, durch Niederalkyl, Niederalkoxy, Halogen, Cyano und/oder Carboxy substituiertes Phenyl oder Oxo substituiertes Niederalkyl; Carboxyl, Niederalkoxycarbonyl, Carbamoyl, Cyano, Niederalkoxy, Halogen, Amino, Niederalkylamino, Diniederalkylamino, Niederalkylamino oder Niederalkanoylamino stehen, m eine ganze Zahl von 1 bis 4 ist, und Salzen von Verbindungen der Formel I.

5. Verfahren zur Herstellung von Verbindung der Formel I gemäss Anspruch 1, worin $R_1$ (1R)-1-Hydroxyäthyl ist, Z für einen Rest

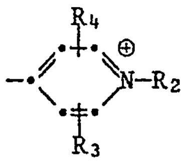

steht, worin $R_2$ Niederalkyl, Acetonyl oder durch Carboxy, Niederalkoxycarbonyl, Carbamoyl, N,N-Diniederalkylcarbamoyl oder Phenyl substituiertes Niederalkyl bedeutet, $R_3$ Wasserstoff bedeutet, $R_4$ für Wasserstoff, durch Hydroxy, carboxy, Cyano oder Amino substituiertes Niederalkyl; Carboxy, Carbamoyl oder Cyano steht, und m 1 oder 2 ist, und Salzen von Verbindungen der Formel I.

6. Verfahren zur Herstellung von Verbindung der Formel I gemäss Anspruch 2, worin $R_1$ Hydroxymethyl oder (1R)-1-Hydroxyäthyl ist, $R_2$ Niederalkyl oder durch Carboxy, Niederalkoxycarbonyl, Carbamoyl, Cyano oder Phenyl substituiertes Niederalkyl bedeutet, $R_3$ Wasserstoff bedeutet, $R_4$ für Wasserstoff, durch Carboxy, Niederalkoxycarbonyl oder Cyano substituiertes Niederalkyl; Carboxy, Niederalkoxycarbonyl oder Cyano steht, und m 1 oder 2 ist, und Salzen von Verbindungen der Formel I.

7. Verfahren zur Herstellung von Verbindung der Formel I gemäss Anspruch 1, worin $R_1$ (1R)-1-Hydroxyäthyl ist, Z für einen Rest

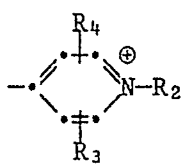

steht, worin $R_2$ Niederalkyl oder durch Carbamoyl substituiertes Niederalkyl ist, $R_3$ Wasserstoff ist und $R_4$ Wasserstoff oder durch Hydroxy substituiertes Niederalkyl bedeutet und m 1 ist, und Salzen von Verbindungen der Formel I.

8. Verfahren zur Herstellung von Verbindung der Formel I gemäss Anspruch 2, worin $R_1$ (1R)-1-Hydroxyäthyl ist, $R_2$ Niederalkyl oder Phenylniederalkyl ist, $R_3$ und $R_4$ Wasserstoff bedeuten und m 1 ist, und Salzen von Verbindungen der Formel I.

9. Verfahren zur Herstellung von (5R,6S)-6-[(1R)-1-Hydroxyäthyl]-2-[(1-methyl-4-pyridinio)-aminomethyl]-2-penem-3-carboxylat gemäss Anspruch 2.

10. Verfahren zur Herstellung von (5R,6S)-2-[(1-Benzyl-4-pyridinio)-aminomethyl]-6-[1R)-1-hydroxyäthyl]-2-penem-3-carboxylat gemäss Anspruch 2.

11. Verfahren zur Herstellung von (5R,6S)-2-[(1-Aminocarbonylmethyl-4-pyridinio)-aminomethyl]-6-[(1R)-1-hydroxyäthyl]-2-penem-3-carboxylat gemäss Anspruch 2.

12. Verfahren zur Herstellung von (5R,6S)-6-[(1R)-1-Hydroxyäthyl]-2-[(2-hydroxymethyl-1-methyl-4-pyridinio)-aminomethyl]-2-penem-3-carboxylat gemäss Anspruch 1.

13. Verfahren zur Herstellung von (5R,6S)-6-[(1R)-1-Hydroxyäthyl]-2-[(2-carboxy-1-methyl-4-pyridinio)-aminomethyl]-2-penem-3-carboxylat und pharmazeutisch annehmbaren Salzen davon gemäss Anspruch 1.

14. Verfahren zur Herstellung von (5R,6S)-6-[(1R)-1-Hydroxyäthyl]-2-[(2-aminomethyl-1-methyl-4-pyridinio)-aminomethyl]-2-penem-3-carboxylat und pharmazeutisch annehmbaren Salzen davon gemäss Anspruch 1.

15. Verfahren zur Herstellung von (5R,6S)-6-[(1R)-1-Hydroxyäthyl]-2-[(1-acetonyl-4-pyridinio)-aminomethyl]-2-penem-3-carboxylat gemäss Anspruch 1.

16. Verfahren zur Herstellung von pharmazeutischen Präparaten, dadurch gekennzeichnet, dass man eine nach dem Verfahren gemäss Anspruch 1, erhältliche Verbindung der Formel I oder ein pharmazeutisch annehmbares Salz einer solchen Verbindung mit einem pharmazeutisch verwendbaren

Trägermaterial mischt.

17. Verfahren zur Herstellung von pharmazeutischen Präparaten, dadurch gekennzeichnet, dass man eine nach dem Verfahren gemäss Anspruch 2 erhältliche Verbindung der Formel I oder ein pharmazeutisch annehmbares Salz einer solchen Verbindung mit einem pharmazeutisch verwendbaren Trägermaterial mischt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 87 81 0462

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A - 0 109 362 (CIBA-GEIGY) * Seite 5, Abstaz 2; Ansprüche * | 1,17, 19 | C 07 D 499/00<br>A 61 K 31/43//<br>C 07 D 213/24 |
| A | GB - A - 2 118 181 (FARMITALIA CARLO ERBA) * Ansprüche * | 1,17, 23 | |
| A | GB - A - 2 042 515 (BRISTOL-MYERS) * Ansprüche * | 1,17 | |
| | | | **RECHERCHIERTE SACHGEBIETE (Int. Cl.4)** |
| | | | C 07 D 499/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-20,23-26

Unvollständig recherchierte Patentansprüche:

Nicht recherchierte Patentansprüche: 21,22

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 19-11-1987 | CHOULY |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82